# EUROPEAN PATENT SPECIFICATION

(11) **EP 3 823 578 B1**
(45) Date of publication and mention of the grant of the patent: **26.08.2026**
(21) Application number: 19740552.5
(22) Date of filing: 15.07.2019
(51) Int. Cl.: A61G 13/10, A61G 13/12

(54) **A FIRST BODY MEMBER OF A JOINT ASSEMBLY RELEASABLY CONNECTING TO AN EXTENSION PANEL TO A PATIENT SUPPORT PANEL MOUNTED ON A PEDESTAL**
EIN ERSTES KÖRPERTEIL EINER VERBINDUNGSANORDNUNG WELCHE LÖSBAR VERBINDET MIT EINER VERLÄNGERUNGSPLATTE MIT EINER AUF EINEM SOCKEL MONTIERTEN PATIENTENLAGERUNGSPLATTE
PREMIER ÉLÉMENT DE CORPS D'UN ENSEMBLE D'ARTICULATION RELIER DE MANIÈRE AMOVIBLE UN PANNEAU D'EXTENSION À UN PANNEAU DE SUPPORT DE PATIENT MONTÉ SUR UN SOCLE

(30) Priority: 16.07.2018 BE 201805513
(43) Date of publication of application: 26.05.2021
(73) Proprietor: Orfit Industries, 2110 Wijnegem (BE)
(72) Inventor: DE GRUYTERE, Simon, 2450 Meerhout (BE); CUYPERS, Steven, 2970 's Gravenwezel (BE); WELLEMAN, Vincent, 2600 Berchem (BE)
(74) Representative: Gevers Patents
(86) International application number: PCT/EP2019/069037
(87) International publication number: WO 2020/016189

(56) References cited:
- JP-A- H0 531 145
- US-A1- 2007 107 129
- US-A1- 2014 068 864
- US-B2- 6 739 006
- US-B2- 8 997 281

## Description

### Technical Field

The present invention relates to a first body member of a joint assembly releasably connecting an extension panel to a patient support panel mounted on a pedestal. In particular the present invention relates to a first body member of a joint assembly which makes it easier to install a patient support assembly comprising an extension panel and a patient support panel for supporting and preferably immobilizing a patient for example in radiation therapy. The invention also relates to the joint assembly for releasably connecting an extension panel to a patient support panel mounted on a pedestal, the joint assembly comprising the first body member. The invention also relates to the extension panel comprising the first body member as well as to the patient support assembly comprising the extension panel.

### State of the art

The present invention deals with patient support assemblies for medical use, such as for radiation therapy or radiation diagnostics for example used with linear accelerators in the treatment of cancers. Patient support assemblies in this technical field are subject to multiple stringent constraints regarding amongst others radiolucency and load supporting capacities. The implementation of these constraints is burdened by the additional requirement of case specificity, as the patient support assemblies have to be specifically designed for a given treatment or diagnosis. It is for example known to provide patient support assemblies comprising a lateral cavity member when performing a lithotripsy treatment. In a different case, such as in radiotherapy, the patient support assembly has to be provided with means for immobilizing a patient such as attachment means for attaching an immobilization mask. In order to conform to the case-specificity constraint, there is thus a need for a modular patient support assembly, comprising a relatively short patient support panel mounted on a pedestal and a relatively long extension panel releasably connectable to the patient support panel. The extension panel can be provided with case specific features such as the attachment means in case of a radiotherapy treatment. Providing such a modular patient support assembly conforming to the above mentioned stringent constraints for example of load support and radiolucency is not an easy task. It is for example not allowable to provide load bearing beams into the extension panel as these beams tend to influence the patient treatment, for example tend to scatter and attenuate radiation in radiation treatment and tend to disrupt the homogeneity of the extension panel, certainly when metallic beams are used. The state of the art therefore provides specific patient support assemblies wherein a joint assembly is provided for coupling the extension panel to the patient support panel, wherein the joint assembly comprises a first body member coupled to the extension panel and a second body member coupled to the patient support panel. Such a first body member, joint assembly, extension panel and patient support assembly are known from EP2852325. This document of the state of the art discloses a first body member of a joint assembly for releasably connecting an extension panel to a patient support panel mounted on a pedestal, the first body member configured to be coupled to the extension panel and wherein the joint assembly comprises a second body member configured to be coupled to the patient support panel, wherein the first body member comprises a tab member delimited by an upper tab member surface and a lower tab member surface extending in an engagement direction between a tab member base end and a tab member free end, and wherein the second body member comprises a cavity member delimited by an upper cavity member surface and a lower cavity member surface extending in the engagement direction between a cavity member closed end and a cavity member open end. The first body member of the state of the art comprises a latch, and the second body member comprises an attachment member, wherein the first body member is arranged to be brought into engagement with the second body member, by moving the cavity member and the tab member with respect to each other and by latching the attachment member with the latch. The first and second body members of the state of the art are designed such that the cavity member and the tab member have to be moved with respect to each other by rotating the tab member along a rotation axis which lies perpendicular to the engagement direction and which goes through the tab member free end, up to a locking position where the latch latches on the attachment member.

The patient support assemblies from the state of the art, although resolving many of the above mentioned constraints regarding for example radiolucency, are not ideal for daily use. As presented above, the patient support assemblies are preferably case specific, and are thus often adapted, for example prior to every procedure, by removing the extension panel and installing a new extension panel. The removal and installation of the extension panels from the patient support panel is a cumbersome process as the first body member coupled to the extension panel has to be engaged with the second body member coupled to the patient support panel. In this engagement process the tab member had to be inserted into the cavity member, which is a tedious process, requiring the nurse and the technical assistant to be deeply focused. In the state of the art however, a final rotation is required in order to bring the tab member and cavity member in the locking position and thus in order to engage the first body member and the second body member. The final rotation imposed by the patient support assembly in the state of the art requires thus that the first body member and second body member are moved towards each other in an angled manner up to the final rotation. As stated above, the extension panel is often relatively long compared to the patient support panel, which makes the movement under and angled position even more difficult as a permanent moment has to be applied by the nurse or technical assistant during the installation. This makes the installation more difficult and tends to create health issues for the nurse and technical assistant. Alternative examples of joint assemblies are known from US2007/107129A1, US8997281B2, JPH0531145A, US2014/068864A1, and US6739006B2.

### Detailed description of the invention

It is a goal of the present invention to provide a new first body member of a joint assembly releasably connecting an extension panel to a patient support panel mounted on a pedestal, wherein the problem encountered in the state of the art is solved. The present invention therefore provides an extension panel releasably connecting to a patient support panel mounted on a pedestal for example along the length direction of the panels, according to the first claim. The first body member is configured to be coupled to the extension panel, and the joint assembly comprises a second body member configured to be coupled to the patient support panel. The first body member comprises one of a cavity member delimited by an upper cavity member surface and a lower cavity member surface extending in an engagement direction, for example the length direction of the panels, between a cavity member closed end and a cavity member open end, and a tab member delimited by an upper tab member surface and a lower tab member surface extending in the engagement direction, for example the length direction of the panels, between a tab member base end and a tab member free end. The second body member comprises the other one of the cavity member and the tab member. Thus if the first body member comprises the tab member, the second body member comprises the cavity member and vice versa. The terms upper and lower herein for example relate to the thickness of the tab member and body member, as well as to the thickness of the extension and patient support panels. The first body member further comprises one of a latch and an attachment member, and the second body member comprises the other one of the attachment member and the latch. Thus if the first body member comprises the latch, the second body member comprises the attachment member and vice versa. The first body member is arranged to be brought into engagement with the second body member, by moving the cavity member with respect to the tab member, for example moving the members towards each other for example by moving one of the members towards the other one of the members, and by latching the attachment member with the latch. According to the present invention, the cavity member is moveable over the tab member by means of a translation movement in the engagement direction up to a locking position where the latch latches on the attachment member. The cavity member being moveable over the tab member means that the tab member and the cavity member can be moved towards each other for example with the tab member moving within a fixed cavity member, or a cavity member moving over a fixed tab member for example with the cavity member enclosing the tab member, or the tab member and the cavity member both being moveably arranged. By preference the first body member coupled to the extension panel, irrespective if provided with the tab member or the cavity member, is arranged moveably, and the second body member coupled to the patient support panel is arranged in a fixed manner.

The first body member of the present invention ensures that the final step of the installation, meaning the final step in the engagement of the first body member and the second body member, which is terminated by the latch latching the attachment member, is a pure translation movement of the cavity member with respect to the tab member and thus of the first body member with respect to the second body member, and thus of the extension panel with respect to the patient support panel. This has the advantage that the installation of the patient support assembly is greatly simplified, as it is no longer required that the nurse or technical assistant move the extension panel in an angled manner up to a point where it can undergo a final rotation in order to bring the assembly in a locking position. This makes the installation easier and additionally ensures an ergonomic design that greatly increases the working conditions of the nurses and technical assistants.

According to an embodiment of the present invention the first body member comprises the cavity member. In an embodiment, the upper tab member surface supports the upper cavity member surface, when the cavity member is translated over the tab member. The embodiment is particularly advantageous when the first body member coupled to the extension panel is moveably arranged, while the second body member coupled to the patient support panel is arranged in a fixed manner. The embodiment has the advantage that the nurse or technical assistant upon inserting the tab member into the cavity member can rest the upper cavity surface on the upper tab member surface and can subsequently rely on the supporting action of the upper tab member surface to carry part of the load of the extension panel when moving the tab member and the cavity member with respect to each other, for example by sliding the cavity member over the tab member up to the locking position. In an embodiment of the present invention the upper tab member surface and the upper cavity member surface are flat surfaces. The advantage of being able to slide the cavity member over the tab member is particularly present when the upper tab member surface and the upper cavity member surface are flat, as this facilitates the sliding movement. It is noted that the present embodiments have been described with the first body member being moveable and the second body member being fixed and with the first body member comprising the cavity member. The present embodiments however can be easily adapted for example in case the moveable first body member comprises the tab member. In such a case, the lower cavity surface provided in the second body member would support the lower tab member surface provided in the first body member, and by preference the lower tab member surface and lower cavity member surfaces are flat surfaces.

In an embodiment of the present invention the upper cavity member surface is provided with a cavity locking member comprising one of a protrusion and a slot, and wherein the upper tab member surface is provided with a tab locking member comprising the other one of the slot and the protrusion, and wherein, upon the cavity member being moved with respect to the tab member, the cavity and tab locking members engage each other. The engagement of the cavity and tab locking members provides a security to the patient support assembly, ensuring that the first and second body members do not disengage in an unprompted manner. In an embodiment, the cavity and tab locking members engage each other by the insertion of the protrusion into the slot. If the first body member and second body member after installation, would move away from each other in an unprompted manner, the protrusion would hit the edge of the slot thereby avoiding the total disengagement of the first and second body members. The embodiment is particularly advantageous when the first body member is provided with the cavity member, as in that case, the upper cavity member surface is supported by the upper tab member surface. In the alternative embodiment where the first body member is provided with the tab member, the cavity locking member and tab locking member are preferably provided in respectively the lower cavity member surface and the tab member lower surface, as in that case the tab member lower surface is supported by the lower cavity surface. In an embodiment, the engagement of the cavity and tab locking members require the lifting, for example the upward rotation or the upward translation or the approaching in an angled or raised manner, of one of the body members with respect to the other one of the body members. For example, in the case where the first body member is moveably arranged and provided with the cavity member, the first body member will have to be lifted with respect to the second body member in order to engage the cavity and tab locking members. In an embodiment, the cavity locking member is a protrusion provided in proximity to the cavity member closed end, for example closer to the cavity member closed end than to the cavity member open end, and wherein the tab locking member is a slot provided in proximity to the tab member free end, for example closer to the tab member free end than to the tab member base end. The embodiment is particularly advantageous in the case where the moveable first body member is provided with the cavity member, where providing the protrusion in proximity of the cavity member closed end allows the nurse or the technical assistant to position, upon insertion of the tab member into the cavity member, the upper cavity member surface on the upper tab member surface and to slide, for example almost effortlessly, the cavity member towards the tab member over a substantial distance of the cavity member surface until the protrusion hits the tab member free end. Upon hitting the protrusion on the tab member free end, the first body member must be lifted with respect to the second body member, for example slightly rotated along an axis lying perpendicular to the engagement direction and lying in the cavity member free end such as to lift the protrusion over the upper tab member surface and into the slot. In an embodiment of the present invention the slot extends in the engagement direction and has a length Lₛₗₒₜ measured in the engagement direction and the protrusion has a length Lₚᵣₒₜᵣᵤₛᵢₒₙ measured in the engagement direction and Lₛₗₒₜ is greater than Lₚᵣₒₜᵣᵤₛᵢₒₙ. Providing Lₛₗₒₜ greater than Lₚᵣₒₜᵣᵤₛᵢₒₙ allows the translation of the tab member within the cavity member with the cavity and tab locking members engaged. By preference Lₛₗₒₜ is sufficiently greater than Lₚᵣₒₜᵣᵤₛᵢₒₙ such as to allow, upon the tab and cavity locking members being engaged, the translation movement of the cavity member with respect to the tab member up to the locking position. In an embodiment of the present invention, the slot has a slot depth Hₛₗₒₜ, and the protrusion has a protrusion height Hₚᵣₒₜᵣᵤₛᵢₒₙ, and wherein Hₛₗₒₜ is greater than Hₚᵣₒₜᵣᵤₛᵢₒₙ. By preference the protrusion and the slot are therefore shaped such that the protrusion fits for example entirely into the slot. Providing Hₛₗₒₜ is greater than Hₚᵣₒₜᵣᵤₛᵢₒₙ enables to create a close sliding fit between the tab member and cavity surfaces provided with the respective tab and cavity locking members, for example the tab member and upper cavity member surface or the tab member and lower cavity member surface. Such a close sliding fit is a fitting wherein the surfaces are adjacent, for example in contact, but still allow to be moved with respect to each other by sliding. Providing such a fit avoids the entrance of pollutants such as dust, blood or chemicals between the surfaces, which enables to maintain a higher degree of cleanness as required for patient support assemblies in the medical field. Furthermore, such a close fit such as a close sliding fit or a substantially snug fit provides a large load and moment bearing area, which would otherwise be carried by a relatively small area of the protrusion contacting the slot.

According to an embodiment of the present invention the tab member and cavity member comprise a part of finite length wherein the upper tab member surface lies substantially parallel, for example parallel, to the lower tab member surface and wherein the upper cavity member surface lies substantially parallel, for example parallel, to the lower cavity member surface. As well the tab or cavity member provided in the first body member as the other one of the cavity or tab member provided in the second body member comprise the part of finite length. In an embodiment the part of finite length comprises a supporting segment. The supporting segment is defined as a segment of the part of finite length along which, upon the first body member being engaged with the second body member, in particular upon the tab member and the cavity member being in the locking position, the upper cavity member surface and the lower cavity member surface are substantially parallel, for example parallel, by preference adjacent to respectively the upper tab member surface and lower tab member surface. Along the supporting segment, the upper cavity member surface and the lower cavity member surface by preference create a close sliding fit with respectively the upper tab member surface and lower tab member surface. In a further embodiment a substantial snug fit is created along the supporting surface. Providing the supporting segment has the advantage that a good load bearing, in particular a good moment bearing connection is provided. Thereto the supporting segment provides a large area of contact between the upper cavity member surface and the upper tab member surface and/or between the lower cavity member surface and the lower tab member surface. Furthermore, providing a close fit such as a close sliding fit or a substantial snug fit alleviates the risk of pollutants entering the cavity member. Additionally, providing a supporting segment forces a translation movement, for example up to a locking position, between the cavity member and the tab member. The length extending in the engagement direction of the supporting segment is by preference between 1cm and 16cm, by further preference between 1cm and 5 cm. In an embodiment of the present invention the length Lₛₗₒₜ is at least equal to the length of the supporting segment, by preference at least equal to the length of the supporting segment and Lₚᵣₒₜᵣᵤₛᵢₒₙ combined. This embodiment ensures that the tab member and cavity member can be translated up to the locking position, when the cavity and tab locking members are engaged. In an embodiment of the present invention the tab member is tapered in the thickness direction increasing between the tab member free end and tab member base end. Preferably the upper tab member surface thereby remains a flat surface. Providing a tapered part on the tab member allows that the body members are approached in an angled manner, in particular in order to engage the cavity and tab locking members. In an embodiment of the present invention the part of finite length in the tab member and the cavity member are respectively provided at the cavity member open end and the tab member base end, for example in proximity to respectively the cavity member open end and the tab member base end, for example respectively closer to the cavity member open end than the cavity member closed end and closer to the tab member base end than the tab member free end, for example respectively ending at the cavity member open end and the tab member base end. The first body member comprising the cavity member provided with the part of finite length at the cavity member open end and the second body member comprising the tab member provided with the part of finite length at the tab member base end, ensures that a translation motion is performed up to the locking position. In an embodiment of the present invention the tab member is tapered in the thickness direction increasing between the tab member free end, for example the thinnest part of the tab member, and the part of finite length provided at the tab member base end, for example the thickest part of the tab member. The insertion of the tab member into the cavity member is thereby simplified as the part of finite length provided at the tab member base end is the thickest part of the tab member corresponding to the large cavity opening provided at the cavity open end, whereas the tab member free end is the thinnest part of the tab member. The insertion of a thin tab member free end into a large cavity member open end is a simple operation. In an alternative embodiment of the present invention, the part of finite length in the tab member and the cavity member are respectively provided at the cavity member closed end and the tab member free end, for example in proximity to respectively the cavity member closed end and the tab member free end, for example respectively closer to the cavity member closed end than to the cavity member open end and closer to the tab member free end than to the tab member base end, for example respectively ending at the cavity member closed end and the tab member free end. By preference, the tab member is tapered from the part of finite length provided at the tab member free end, for example the thinnest part of the tab member, increasing to the tab member base end, for example the thickest part of the tab member. The length of the supporting segment provided at the cavity member closed end of the cavity member is by preference a substantial part of the length extending in the engagement direction of the part of finite length. By preference, a clearance is left between the tab member free end and the cavity member closed end when the tab member and cavity member are in locking position, such as to take into consideration dimensional changes of the tab member for example due to thermal expansion or wear of the tab member. In an embodiment of the present invention the tab member and cavity member which comprise a first part of finite length respectively provided at the tab member base end and the cavity member open end, comprise a second part of finite length respectively provided at the tab member free end and the cavity member closed end. By preference, the tab member is tapered from the second part of finite length provided at the tab member free end, for example the thinnest part of the tab member, increasing to the first part of finite length provided at the tab member base end, for example the thickest part of the tab member. Providing a first and second part of finite length, by preference both provided with a supporting segment, increases the load and specifically moment bearing capacity of the assembly. By preference the length of the supporting segment provided at the cavity member closed end of the cavity member is by preference a substantial part of the length extending in the engagement direction of the part of finite length, such as to create a clearance between the tab member free end and the cavity member closed end upon the first and second body parts being in the locking position. By preference each of the supporting segments has a length of between 1cm and 16cm, by further preference between 1cm and 5 cm. By preference the length Lₛₗₒₜ is at least equal to the length of the supporting segment having the greatest length, by preference at least equal to the length of the supporting segment having the greatest length and Lₚᵣₒₜᵣᵤₛᵢₒₙ combined.

In an embodiment of the present invention the tab member has a substantially congruent shape to the cavity member.. The tab member comprises two tab member sidewalls which are laterally opposed, Both tab member sidewalls are preferably tapered in the lateral direction along the engagement direction such that the width measured in the lateral direction of the tab member is larger at the tab member base end than at the tab member free end. The cavity member preferably comprises two cavity member sidewalls which are laterally opposed. Both cavity member sidewalls are preferably tapered in the lateral direction along the engagement direction such that the width measured in the lateral direction of the opening of the cavity member is larger at the cavity member open end than at the cavity member closed end. In an embodiment of the present invention the sidewalls of the tab member are congruently shaped to the sidewalls of the cavity member. In an embodiment of the present invention, upon the tab member and the cavity member being engaged, the sidewalls of the tab member lie adjacent to the sidewalls of the cavity member, by preference forming a close sliding fit. This embodiment has the advantage that the cavity members are strongly secured over the tab members such as to hinder, for example substantially prohibit, any lateral movement of the cavity member and the tab member with respect to each other. By preference the tab locking member and the cavity locking member have substantially the same width, thereby additionally hindering, for example substantially prohibiting, any lateral movement of the cavity member and the tab member with respect to each other.

In an embodiment of the present invention one of the first body member and second body member comprises two cavities, and the other one of first body member and second body member comprises two tab members. Providing two tab and cavity members allows to place two tab and cavity members apart over a lateral distance in a lateral direction perpendicular to the engagement direction and lying in respectively the tab member free end and cavity member closed end. Providing the two cavity and tab members separated over a lateral distance increases the load bearing and in particular the moment bearing capacity, in particular the capacity to bear the moment applied to the extension table along the engagement direction, for example applied upon the patient being placed substantially on one lateral side of the extension table, for example upon rolling the patient on its side. In a further embodiment, more than two cavity and tab members are provided. In a further embodiment, the first body member comprises a tab member and a cavity member, and the second body member also comprises a tab member and a cavity member arranged such as to engage the tab and cavity member of the first body member. In an embodiment, the latch and attachment member are positioned on an intermediate position between one of the two cavities and the two tab members. Preferably the latch is provided on the intermediate position between two tab members, such that upon falling, the tab members protect the latch from damage by substantially absorbing the shock.

In an embodiment of the present invention, the cavity member is formed by an upper cavity plate and a lower cavity plate, wherein the upper cavity surface and lower cavity surface are the inner surfaces of respectively the upper cavity plate and the lower cavity plate. In an embodiment of the present invention the upper cavity plate outer surface is part of the extension panel outer surface, for example the surface on which the patient is positioned.

In the further embodiments, the latch and attachment member will be further exemplified. In an embodiment of the present invention the first body member is provided with the attachment member. In this embodiment the second body member is provided with the latch. Providing the first body member with the latch increases the risk of damaging the assembly. By preference the latch is a moveable part, for example extending from the body member on which it is provided. Upon dropping the body member on which the latch is provided, the latch has a risk of being damaged. By providing the latch on the second body member, which is less subject to movement with respect to the exchangeable extension panel, the risk of damaging the latch is drastically decreased. Furthermore the latch is preferably provided into a hole provided in the body member provided with the latch, such as to decrease the risk of damaging the latch. The attachment member is in that case provided such as to extend from the body member provided with the attachment member. Alternatively, the attachment member is provided into a hole, whereas the latch extends from the body member or is partially provided into a hole and extends partially from the body member. Preferably the latch is provided on the body member comprising the tab member, such that the tab member absorbs the shock upon falling rather than the latch. The attachment member is preferably a protrusion onto which the latch can latch. The latch is thereto moveable in a latching direction between an unlatched state and a latched state, preferably by rotation of the latch around a latch rotation axis, preferably parallel to the thickness direction of the panels. Preferably the protrusion of the attachment member extends in a direction comprising a component parallel to the direction of the latch rotation axis, preferably extends in a direction parallel to the direction of the latch rotation axis. The protrusion of the attachment member is preferably connected at its both ends to the body member comprising the attachment member, thereby alleviating the risk that the latch would be unwantedly unlatched from the protrusion of the attachment member for example by the sliding of the latch along the protrusion up to a protrusion free end. In an embodiment of the present invention the latch comprises an abutment part and a latching part, wherein, upon the cavity member being translated over the tab member towards the tab member base end, the attachment member abuts the abutment part causing, for example by the attachment member pushing the abutment member, the latch to switch from an unlatched state to a latched state wherein the latching part latches on to the attachment member. The latch is preferably biased to be in the unlatched state, for example by the permanent action of a spring forcing the latch into the unlatched state. Due to the latch being biased in the unlatched state, upon the cavity member being translated over the tab member towards the tab member free end, the attachment member preferably releases the abutment part causing the latch to switch from the latched state to the unlatched state. The embodiments have the advantage that the latch latches automatically upon the nurse or technical assistant translating the tab member and cavity member towards each other into the locking position. No further actions are required by the nurse of technical assistant, which implies that they could install the assembly on their own, as a single person. In an embodiment of the present invention the latch comprises a pivot pin, anchoring the latch to the body member comprising the latch, with a rotational degree of freedom, for example around the latch rotation axis. In the embodiment, upon the attachment member abutting the abutment part, the latch rotates around the pivot point, for example due to the attachment member pushing on the abutment part thereby applying a moment on the latch, causing the latching part to latch on to the attachment member. The abutment part is thereto preferably provided with an abutment surface which is angled with respect to the engagement direction certainly when the attachment part and the pivot pin are not separated along the lateral direction perpendicular to the engagement direction and the direction of the latch rotation axis. In an embodiment of the present invention the body member comprising the latch, comprises a latch locking device which is brought from an unlocked state to a locked state when the latch is brought into the latched state, for example as a reaction of the movement of the latch from the unlatched state towards the latched state for example upon the latch reaching the latched state. The latch locking device in the locked state prevents the latch from being brought into the unlatched state. Upon the latch locking device being in the locked state, the latch is bound to remain in the latched state. The present embodiment substantially increases the security of the patient support assembly. Furthermore, the present embodiment has the advantage that the latch locking device automatically locks the latch in the latched position upon the nurse or technical assistant translating the tab member and cavity member towards each other into the locking position. No further actions are required by the nurse of technical assistant, which implies that they could install the assembly on their own, as a single person. In an embodiment of the present invention the latch locking device is brought in the locked state by the rotation of the latch around the pivot point, for example upon the attachment member applying a moment on the latch by pushing the abutment part of the latch. In an embodiment of the present invention the latch locking device comprises a locking pin and the latch comprises a locking hole, and the locking pin is inserted into the locking hole when the latch is brought into the latched state for example such as to bring the latch locking device into the locked state. More specifically, the latch locking device is brought into the locked state because, upon the latch moving from an unlatched state to a latched state, more particularly upon the latch arriving in the latched state, the locking pin is inserted into the locking hole. The latch locking device, in particular the locking pin is thereto biased to be in the locked state, for example the locking pin is permanently pushed against the latch for example under influence of gravitational force acting on the locking pin or under influence of a spring pushing the locking pin. The locking pin is thereto moveably arranged in the direction along which the biasing force is applied, for example perpendicular on the latch surface, by preference along the direction of the latch rotational axis. The latch is thereto moveable in a direction crossing the biasing force direction of the locking pin, for example movable in a plane perpendicular to the biasing force direction, for example along the latching direction caused by the rotation of the latch around the latch rotation axis. The latch is provided with the locking hole at a specific position such that when the latch is in the latched state, the position of the locking hole along the lateral and engagement directions corresponds to the position of the locking pin. Upon the latch being in the unlatched state, the locking pin is biased to push against the latch at a position different from the position where the locking hole is provided. Upon the latch being brought into the latched position, for example upon the latch arriving in the latched position, the locking pin is inserted, for example pushed, into the locking hole and is kept in the locking hole by the biasing force. In an embodiment of the present invention one of the first body member and second body member comprises a latch actuator configured to, upon actuation, for example manually, bring the latch locking device from the locked state into the unlocked state. In an embodiment the latch actuator is configured to, upon actuation, retrieve the locking pin from the locking hole. The actuation thereto comprises generating a force counteracting the biasing force applied on the latch locking device, for example on the locking pin. In an embodiment of the present invention the actuation of the latch actuator requires the pulling of a handle. By preference the latch actuator, apart from being subjected to the biasing forces on the locking device, is also subjected to biasing forces acting directly on the latch actuator such as gravitational forces or spring forces. By preference the handle is provided in the outer surface of the panel comprising the body member comprising the latch actuator. By preference the handle upon being pulled, extends above the outer surface of the panel comprising the body member comprising the latch actuator. This embodiment increases the security of the system, as the latch actuator can only be actuated, by lifting the handle above the outer surface of the panel, and can thus only be actuated when the patient is not supported on the outer surface of the panel. In an embodiment of the present invention the body member comprising the latch, comprises a moveable plate. The moveable plate is switchable between an opened state and a closed state, wherein the moveable plate in the closed state prevents the latch locking device from being brought in the locked state. The moveable plate is brought into the closed state when the latch locking device is brought in the unlocked state, for example by the actuation of the latch actuator, and the latch is in the latched state. In an embodiment the moveable plate covers the locking hole when the moveable plate is in the closed state, thereby being positioned between the locking hole and the locking pin. In that case, the locking pin is being pressed against the moveable plate by the biasing force acting on the locking pin, thereby preventing the latch locking device from being brought into the locked state and by consequence preventing, without the user having to maintain the latch actuator in the actuated state, the latch actuator from switching back to the non-actuated state. By preference the moveable plate is biased to be in the closed state, for example by a biasing force acting on the moveable plate for example by a spring. The biasing force acting on the moveable plate for example permanently pushes the moveable plate towards a position corresponding to the position of the locking hole in the latch when the latch is in the latched state. By preference both the latch locking device in the locked state, for example with the locking pin inserted into the locking hole as well as the latch in the unlatched state prevent the moveable plate from being brought into the closed state. Both states as previously mentioned prevent the moveable plate from being brought into the closed state independently from each other. Furthermore, upon the latch being brought in the latched state, during the transition, the latch gradually stops blocking the moveable plate from entering the closed state, but simultaneously the locking device, although not yet entirely in the locked state, already starts blocking the movable plate from entering the closed state. It is noted furthermore that the latch locking device cannot be in the locked state when the latch is in the unlatched state. Therefore, the moveable plate can only be brought into the closed state when the latch is in the latched state and upon the latch locking device being brought in the unlocked state. It is noted that the states of the moveable plate are not directly controlled by the user such as the nurse or the technical assistant. The nurse or technical assistant can only control the latch actuator and the translation of the extension panel and patient support panel with respect to each other in order to respectively unlock the latch locking device and to bring the latch into the latched or unlatched states. It is noted that the locking of the locking device, and thus the switching of the actuator to a non-actuated state, is operated by the biasing forces acting on the locking device and follow from the controllable actions of the user. It is furthermore notes that and the unlatching of the latch is facilitated by the biasing forces biasing the latch into the unlatched state. The states of the moveable plate are a consequence of the combined states of the locking device under control of the latch actuator and the latch under control of the user translation. In an embodiment of the present invention the moveable plate in the closed state is brought into the opened state when the latch is brought into the unlatched state. Providing the moveable plate allows to create a patient support assembly having a three-state installation and removal. The patient support assembly can be in any of the following three consecutive states: «unlatched and ready to mount»,, «Latched and locked», and «Latched and ready to remove». The major advantage of these embodiments is that the nurse or technical assistant can unlock the latch locking device by actuating the latch actuator and subsequently, without having to maintain the actuation of the latch actuator, for example without having to maintain a counterforce on the latch locking device to counteract the biasing force on the latch locking device, translate the tab member and cavity member away from each other in order to disengage, for example remove the extension panel from the patient support panel. No combined actions are required by the nurse of technical assistant, such as simultaneously actuating the latch actuator and translating the cavity member with respect to the tab member, which implies that they could install the assembly on their own, as a single person. In an embodiment of the present invention the moveable plate is a swivel plate comprising a pivot pin, anchoring the swivel plate to the body member comprising the latch, with a rotational degree of freedom, for example along a direction parallel to the direction of the latch rotation axis. The biasing force acting on the swivel plate tends to rotate the swivel plate in a position corresponding to the position of the locking hole in the latch when the latch is in the latched state.

Further disclosed is an extension panel comprising the first body member of the joint assembly. By preference the extension panel, and analogously the patient support panel have a length direction along which the extension panel and the patient support panel are releasably connected. By preference the extension panel, and analogously the patient support panel have a thickness direction along the thickness of the panel, extending between a panel upper surface, configured to support the patient, and a panel lower surface. The panel lower surface of the patient support panel is preferably connected to the pedestal. The panels have a lateral direction perpendicular to the length direction and the thickness direction. In an embodiment of the present invention, upon the first body member being engaged with the second body member, the upper surface of the patient support panel lies in plane with, for example substantially flush with, the upper surface of the extension panel. In an embodiment the upper surface of the extension panel and the upper surface of the patient support panel are separated by a margin. In an embodiment the margin is sealed by a seal, for example sealed by one or more seals extending from the extremities of one or both panels along the length direction. Preferably the seal functions as a sealing element alleviating pollutants from entering into for example the cavity member. In a preferred embodiment the seal functions as a shock absorbing element protecting the extremities of the panels upon connection of the panels, for example in addition to the sealing element function. In an embodiment of the present invention the extension panel is a radiolucent panel. This embodiment enables the patient support assembly to be used for radiation therapy or diagnostics

It is a further aim of the present invention to provide a joint assembly for releasably connecting an extension panel to a patient support panel mounted on a pedestal, the joint assembly comprising the first body member configured to be coupled to the extension panel and comprising the second body member configured to be coupled to the patient support panel.

It is the aim of the present invention to provide a patient support assembly comprising the joint assembly, wherein the first body member is coupled to the extension panel and the second body member is coupled to the patient support panel, the latter preferably being provided on the pedestal. In an embodiment the first body member and the second body member are integrally connected, for example non-releasably connected, to respectively the extension panel and the patient support panel.

### Figures

Figure 1 shows a perspective view of the patient support assembly, illustrating the components of the second body member
Figure 2 shows a different perspective view of a part of the patient support assembly of figure 1, illustrating the components of the first body member
Figure 3 shows a cross-sectional view of a part of the patient support assembly of figure 1 along a plane perpendicular to the lateral direction and through a tab and cavity member
Figure 4 shows a cross-sectional view of a part of the patient support assembly of figure 1 along a plane perpendicular to the thickness direction, the patient support assembly being in an unlocked and ready to be mounted state
Figure 5 shows a perspective view of the patient support assembly as shown in figure 4
Figure 6 shows a cross-sectional view of a part of the patient support assembly of figure 1 along a plane perpendicular to the thickness direction, the patient support assembly being in a locked state
Figure 7 shows a perspective view of the patient support assembly as shown in figure 6
Figure 8 shows a cross-sectional view of a part of the patient support assembly of figure 1 along a plane perpendicular to the thickness direction, the patient support assembly being in an unlocked and ready to dismount state
Figure 9 shows a perspective view of the patient support assembly as shown in figure 8
Figure 10 shows a three-stage diagram of the patient support assembly

### Description of the figures

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not necessarily correspond to actual reductions to practice of the invention.

Furthermore, the terms first, second, third and the like in the description and in the claims, are used for distinguishing between similar elements and not necessarily for describing a sequential or chronological order. The terms are interchangeable under appropriate circumstances and the embodiments of the invention can operate in other sequences than described or illustrated herein.

Furthermore, the various embodiments, although referred to as "preferred" are to be construed as exemplary manners in which the invention may be implemented rather than as limiting the scope of the invention.

The term "comprising", used in the claims, should not be interpreted as being restricted to the elements or steps listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a device comprising A and B" should not be limited to devices consisting only of components A and B, rather with respect to the present invention, the only enumerated components of the device are A and B, and further the claim should be interpreted as including equivalents of those components.

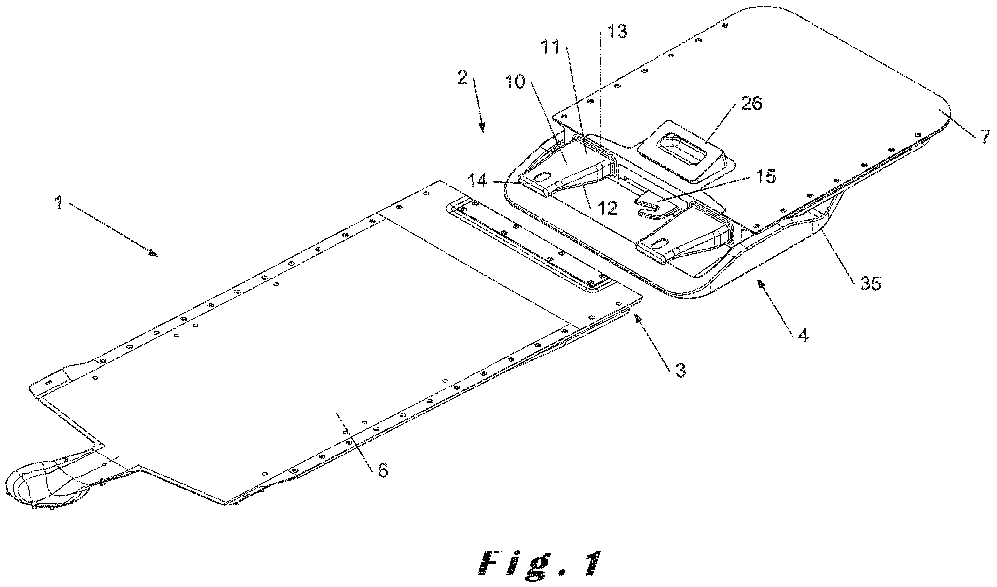

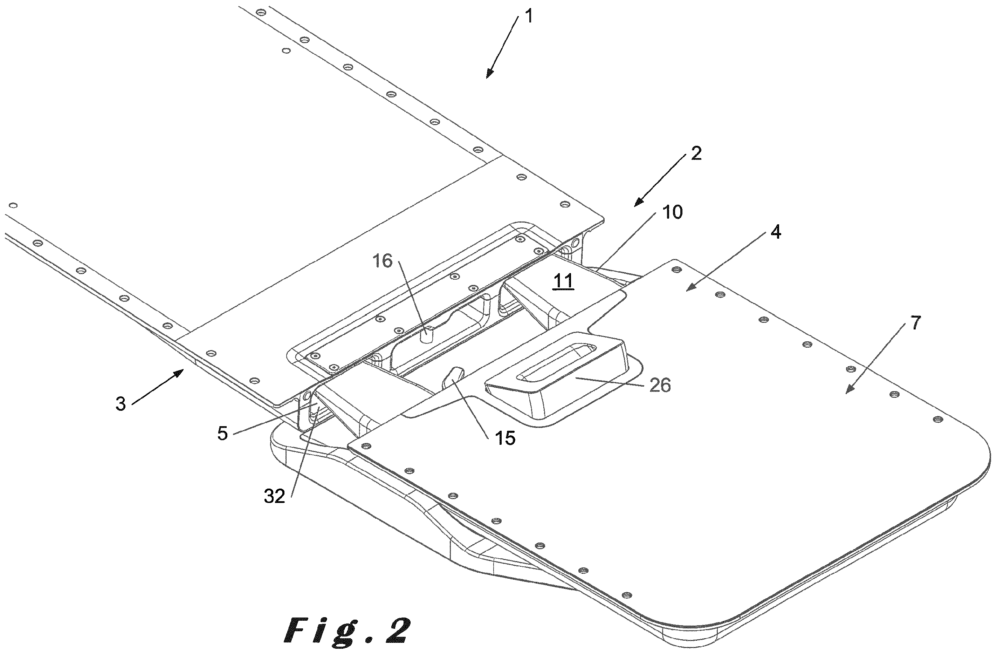

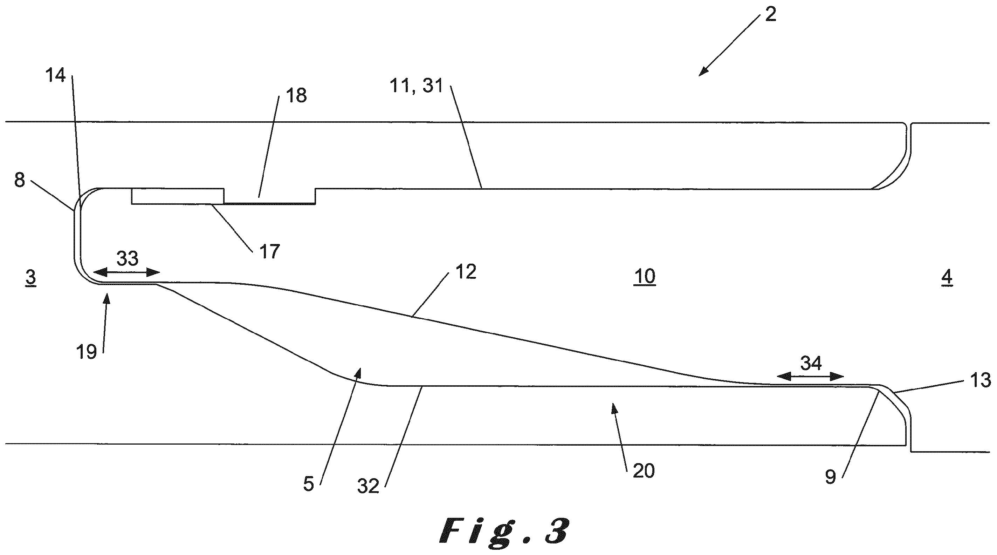

It is an aim of the present invention to provide a first body member of a joint assembly for releasably connecting an extension panel to a patient support panel mounted on a pedestal. In particular the present invention relates to a first body member of a joint assembly which makes it easier to install a patient support assembly comprising an extension panel and a patient support panel for supporting and preferably immobilizing a patient for example in radiation therapy. Figure 1 shows a perspective view of such a patient support assembly 1, specifically illustrating the components of the second body member 4. Specific components of the first body member 3 as well as further components of the second body member 4 are shown in the figures 2 and 3. Figure 1 specifically shows the first body member 3 of a joint assembly 2 for releasably connecting an extension panel 6 to a patient support panel 7 mounted on a pedestal (not shown), the first body member 3 configured to be coupled to the extension panel 6, for example permanently coupled to the extension panel 6. The extension panel 6, for example a radiolucent panel, is provided with holes for the attachment of attachment means such as an immobilization mask. The extension panels 6 in general are modular, meaning that they can be easily replaced by other extension panels 6 specifically designed for a given case such as a specific radiation therapy. The extension panel 6 must therefore be easily connected to and released from the patient support panel 7. The joint assembly 2 of the present invention enables this simplified connection and release, whilst providing a joint assembly 2 which minimally hinders the functionality of the panels such as the radiolucency of the panels, and which simultaneously maximizes the load and moment bearing capacity of the assembly enabling heavy persons to be supported by the extension panel 6 even when they are positioned at an extremity of the extension panel 6. In figure 1 the extension panel 6 and the patient support panel 7 are shown in a released state. The patient support panel 7 is provided on a tray 35, configured to adapt the position of the pedestal with regard to the second body member 4. Figure 1 particularly shows the joint assembly 2 comprising the second body member 4 configured to be coupled to the patient support panel 7, for example permanently coupled to the patient support panel 7. As illustrated in figures 1-3, the second body member 4 is provided with two tab members 10 each delimited by an upper tab member surface 11 and a lower tab member surface 12 extending in the engagement direction between a tab member base end 13 and a tab member free end 14. The tab member 10 has a substantially flat upper tab member surface 11 and a partially tapered lower tab member surface 12. This is shown in more detail in figure 3. Figure 3 further shows that the tab member 10 comprises two parts of finite length 19,20 where the upper tab member surface 11 and the lower tab member surface 12 lie substantially parallel to each other. The tab member 10 comprises a first part of finite length 20 at the tab member base end 13 and a second part of finite length 19 at the tab member free end 14. The lower tab member surface 12 is tapered between the second part of finite length 19 of the tab member 10 which is the thinnest part of the tab member 10, increasing in thickness towards the first part of finite length 20 of the tab member 10 which is the thickest part of the tab member 10. It is furthermore shown that the tab member 10 comprises a tab locking member 17 which is an elongated slot. As illustrated in figures 2 and 3, the first body member 3 comprises complementary features to the second body member 4. It is shown that the first body member 3 comprises two cavity members 5 configured for receiving the two tab members 10, each one of the cavity members 5 delimited by an upper cavity member surface 31 and a lower cavity member surface 32extending in an engagement direction between a cavity member closed end 8 and a cavity member open end 9. The cavity member 5 comprises a first part of finite length 20 at the cavity member open end 9 and a second part of finite length 19 at the cavity member closed end 8. The lower cavity member surface 32 is tapered between the second part of finite length 19 which is the thinnest part of the cavity member 5, for example having the smallest opening, increasing in thickness towards the first part of finite length 20 which is the thickest part of the cavity member 5, for example having the largest opening. It is furthermore shown that the cavity member 5 comprises a cavity locking member 18 which is a protrusion. A part of the first and second parts of finite length 19, 20 are supporting segments, respectively supporting segments 33 and 34. The first supporting segment 34 is the part of the first part of finite length 20 where, upon the tab member 10 and the cavity member 5 being engaged, specifically being in the locking position, the upper tab member surface 11 and lower tab member surface 12 are respectively adjacent, for example in a close sliding fit arrangement, with the upper cavity member surface 31 and the lower cavity member surface 32. As shown in figure 3, this first supporting segment 34 extends in the engagement direction between the cavity member open end 9 and the onset of the tapering of the lower tab member surface 12. The second supporting segment 33 is the part of the second part of finite length 19 where, upon the tab member 10 and the cavity member 5 being engaged, specifically being in the locking position, the upper tab member surface 11 and lower tab member surface 12 are respectively adjacent, for example in a close sliding fit arrangement, with the upper cavity member surface 31 and the lower cavity member surface 32. As shown in figure 3, this second supporting segment 33 extends in the engagement direction between the tab member free end 14 and the onset of the tapering of the lower cavity member surface 32. As shown in figures 1-2, the second body member 4 comprises a latch 15, actuated by a latch actuator 26. The latch 15 extends partly from the second body member 4 and is provided between the tab members 10. As shown in figure 2, the first body member 3 comprises an attachment member 16 at a position corresponding to the latch 15 of the second body member 4, more particularly positioned between the cavity members 5. The attachment member 16 is a protrusion extending in the thickness direction from a lower side of the first body member 3 to an upper side of the first body member 3. The protrusion is attached to the lower and upper sides of the first body member 3 and is provided in a hole in the first body member 3.

Figures 1-3 furthermore show the engagement process of the first body member 3 and the second body member 4. In figure 1 the extension panel 6 and the patient support panel 7 are shown in a released stage, ready to be mounted. In figure 2 cavity members 5 of the first body member 3 and the tab members 10 of the second body member 4 are aligned along the engagement direction. The first body member 3 is moved towards the fixed second body member such as to insert the tab members 10 into the cavity members 5. The upper cavity surface 31 is thereby placed on the upper tab surface 11 for example by a nurse or a technical assistant or for example by a robotic arm. The upper tab surface 11 thus supports the upper cavity surface 31. Subsequently the first and second body members 3,4 are moved towards each other by a translation movement, wherein the cavity members 5 are moved in a sliding motion over the tab members 10, for example by sliding the upper cavity member surface 31 over the upper tab member surface 11, until the cavity locking member 18 abuts the tab member free end 14. The movement is typically done by pushing the extension panel 6 towards the patient support panel 7, for example by manually pushing by a nurse or a technical assistant or for example by automatically pushing by a robotic arm. Upon the cavity locking member 18 abutting the tab member free end 14, the extension panel 6 is lifted, for example tilted, with respect to the patient support panel 7, for example by a nurse or technical assistant or for example by a robotic arm. The tapered shape of the tab member 10 allows the tilting movement. Subsequently, the cavity locking member 18 is introduced into the tab locking member 17 by advancing the extension panel 6 with respect to the patient support panel 7 in an angled manner over a minor distance and by dropping, for example un-tilting, the extension panel 6 with respect to the patient support panel 7. Finally, the tab member 10 is moved within the cavity member 5 by means of a translation movement in the engagement direction up to a locking position where the latch 15 latches on the attachment member 16 for example by a nurse or technical assistant or for example by a robotic arm, pushing the extension panel 6 towards the patient support panel 7. This final translation movement is made possible by providing the elongated slot as the tab locking member 17, wherein the protrusion of the cavity locking member 18 can translate. The patient support assembly 1 of the present invention, when provided with tab and cavity locking members 17, 18, merely requires a single lifting of the extension panel 6 with respect to the patient support panel 7 and a movement in an elevated manner, for example an angled manner over a minor distance followed by dropping operation in order to engage the locking members 17, 18. The subsequent movement of the extension panel 6 and the patient support panel 7 is simplified by the translation of the extension panel 6 towards the patient support panel 7 for example by the supporting action of the upper tab surface 11 on the upper cavity surface 31. Patient support assemblies 1 of the state of the art however, when provided with tab and cavity locking members 17,18, require at least one lifting and one tilting operation, for example two tilting operations, of the extension panel 6 with respect to the patient support panel 7, for example one operation in order to engage the locking members 17,18 followed by the dropping of the extension panel 6 with respect to the patient support panel 7 and one operation for the final rotation into the locking position. Alternatively, the patient support assemblies 1 of the state of the art, when provided with tab and cavity locking members 17, 18 require a single lifting operation, for example a single tilting operation followed by a complex movement of the extension panel 6 towards the patient support panel 7 over a large distance, for example in an angled manner, up to the position where the extension panel 6 undergoes final rotation into the locking position.

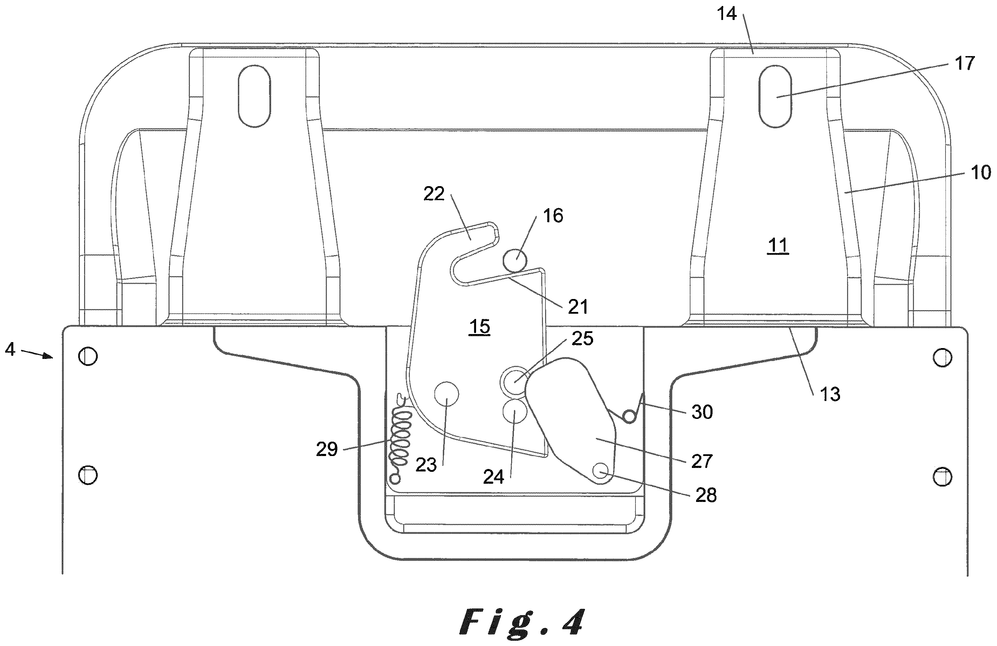

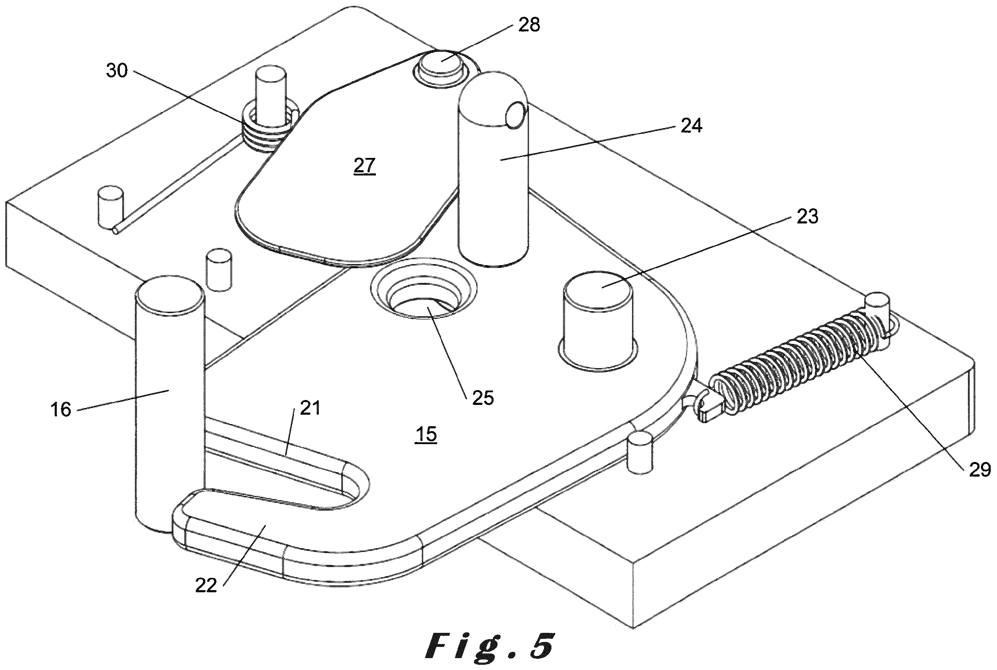

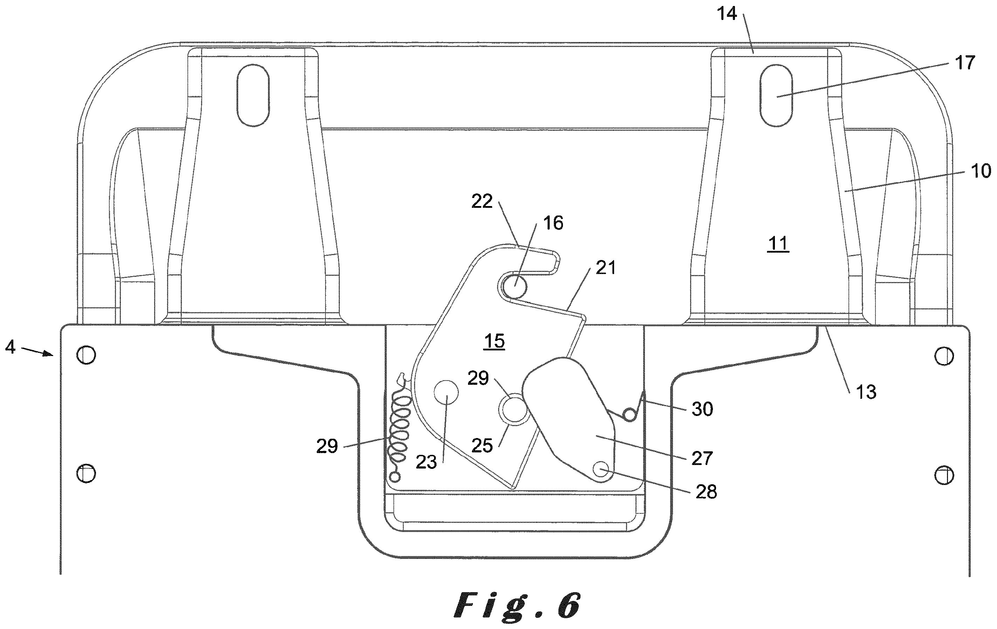

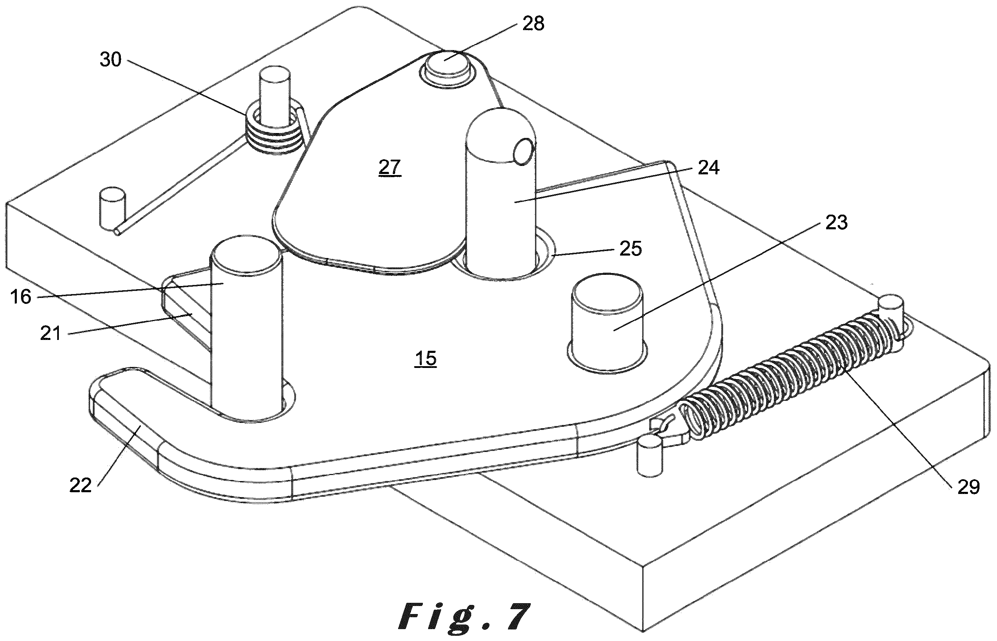

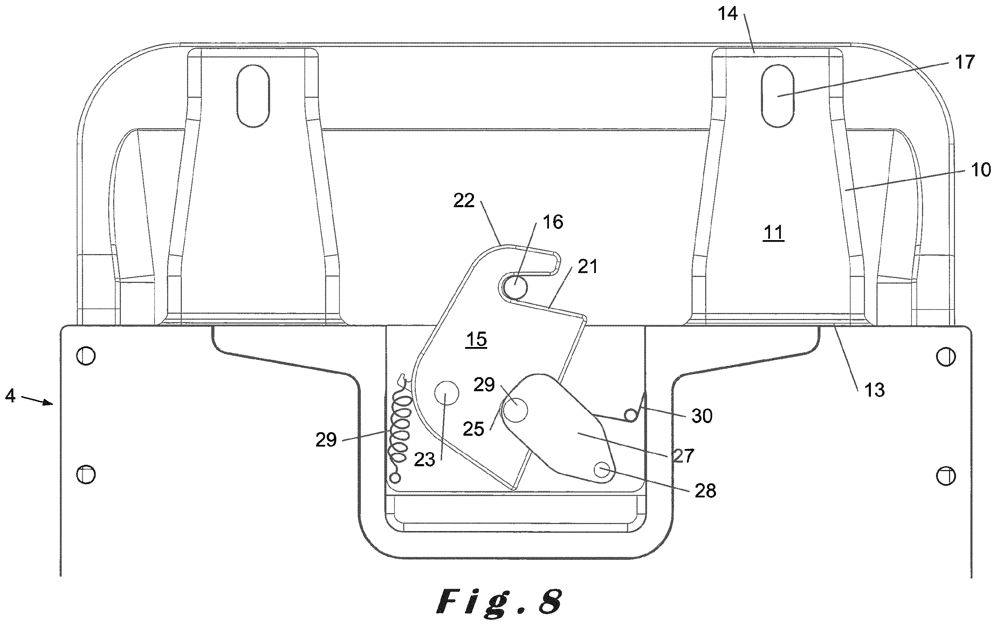

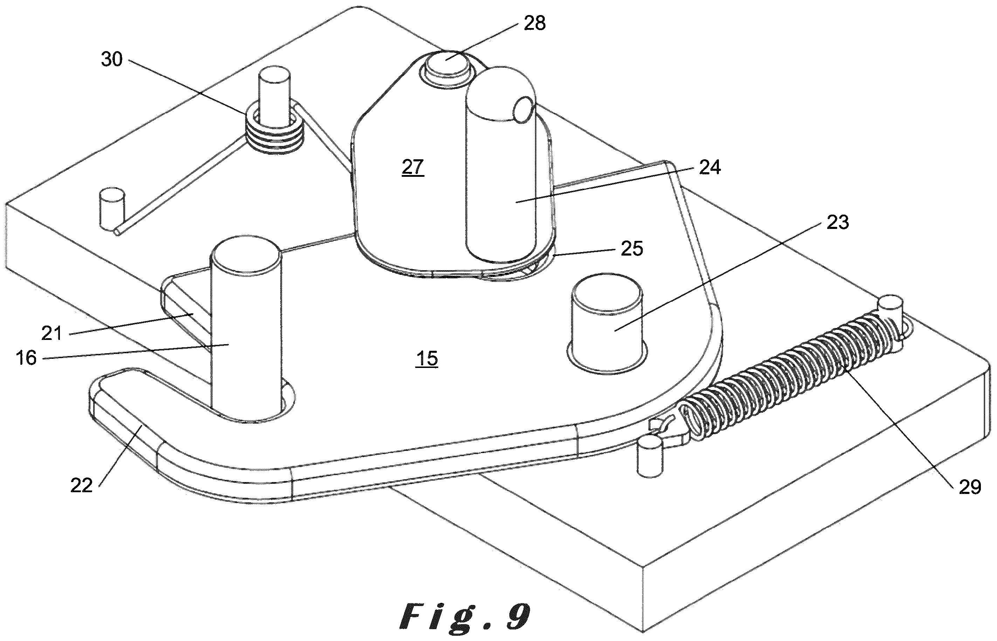

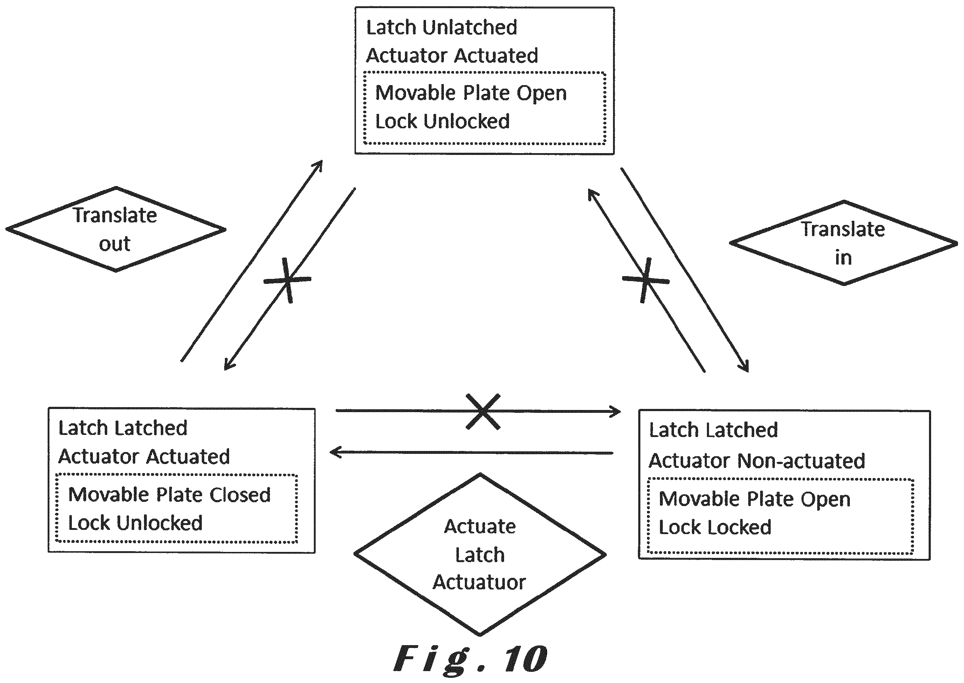

The figures 4 - 9 illustrate in more detail the working of the latch 15 and abutment member 16 upon engagement of the first and second body members 3, 4. Figure 10 is a machine state diagram showing the three consecutive stages in which the patient support assembly 1 can occur due to the working of the latch 15 and attachment member 16, namely «unlatched and ready to mount», «Latched and locked», and «Latched and ready to remove». The three patient support assembly 1 stages are defined by a combination of the states of the latch actuator 26 and the latch 15, each comprising two states, in particular in the order logical 1 and logical 0, namely respectively latch actuator non-actuated state or latch actuator actuated state and latched state or unlatched state. It is noted that the combination of an unlatched state and latch actuator non-actuated state is physically not possible and therefore does not yield a patient support assembly 1 stage. It is furthermore noted that the movable plate 27 can also occur in two states, in particular respectively logical 1 and logical 0, namely a closed state or an opened state. Furthermore, the locking device 24 can also be in two states, in particular respectively logical 1 and logical 0, namely locked state and unlocked state. The states of the movable plate 27 and the states of the locking device 24 are referred to as dependent states, as they are linearly dependent on the combination of the states of the latch actuator 26 and the latch 15, referred to as the variable states. More specifically, the movable plate 27 is in a logical-AND relationship with the variable states and the locking device 24 is in a logical-NOT relationship with the states of the latch actuator 26. Figure 10 shows for every patient support assembly 1 stage the associated variable states is the solid line box and the corresponding dependent states in the dashed box inside of the solid line box. Figure 10 furthermore shows the transitions between the three stages of the patient support assembly 1, which transitions are made possible by exerting a single control action such as the translation of the extension panel 6 up to the locking position, the translation of the extension panel 6 away from the locking position or the actuation of the latch actuator 26. It is noted that the user such as the nurse can only perform the above mentioned control actions. It is furthermore noted that the switching of the latch actuator 26 from the actuated state to the non-actuated state is performed by the biasing forces on the latch actuator 26 as a consequence of the control action wherein the user translates the extension panel 6 up to the locking position. The figures 4, 6 and 8 mainly show the components of the second body member 4 such as the latch 15 and the tab members 10, specifically the upper tab member surface 11 extending between the tab member free end 14 and the tab member base end 13, provided with the tab locking member 17. The only component shown of the first body member 3 in figures 4-9 is the attachment member 16. The second member 4 is furthermore provided with specific components to interact with the attachment member 16 such as to bring the patient support assembly 1 in the three stages as shown in figure 10. The further specific components of the second body member 4 comprise details of the latch 15, a moveable plate configured as a swivel plate 27, a locking device 24 and biasing springs 29, 30. The latch 15 specifically comprises an abutment part 21 and a latching part 22. The latching part 22 and the abutment part 21 extend from the second body member 4. The other above mentioned further components of the second body member 4 are provided in a hole in the second body member 4. The latch 15 further comprises a locking hole 25 and a biasing spring 29. The latch 15 is arranged with a rotation degree of freedom around a pivot pin 23. The locking device 24 comprises a locking pin which is moveably arranged along the thickness direction, substantially along the gravitational acceleration vector. The locking pin 24 is therefore biased against the latch 15. Finally, the second body member also comprises a swivel plate 27 rotationally arranged around a pivot pin 28. The swivel plate 27 is biased by a biasing spring 30. Figure 4 shows a cross-sectional view of a part of the patient support assembly 1 of figure 1 along a plane perpendicular to the thickness direction, the patient support assembly being in an unlocked and ready to be mounted stage. Figure 5 shows a perspective view of the patient support assembly as shown in figure 4. Upon the cavity member 5 being translated over the tab member 10 towards the tab member base end 13, the attachment member 16 abuts the abutment part 21 causing, by the attachment member 16 pushing the abutment member 21, the latch 15 to switch from an unlatched state to a latched state wherein the latching part 22 latches on to the attachment member 16. The latch is biased to be in the unlatched state by the permanent action of biasing spring 29 forcing the latch 15 into the unlatched state. The pushing action of the attachment member 16 against the abutment member 21, for example by a nurse or a technical assistant pushing the extension panel 6 towards the patient support panel 7, counteracts the biasing force of the biasing spring 29, thereby bringing the latch 15 from the unlatched state to the latched state. More particularly, upon the attachment member 16 abutting the abutment part 21, the latch 15 rotates around the pivot point 23 due to the attachment member 16 pushing on the abutment part 21 thereby applying a moment on the latch 15, causing the latching part 22 to latch on to the attachment member 16. The latch locking device, specifically the locking pin 24, presses against the latch 15 at a position different from where the locking hole 25 is provided. The locking device 24 is therefore in an unlocked state. As long as the latch is in the unlatched state, as shown in figures 4 and 5, the locking device 24 cannot be in a locked state, being a state wherein the locking pin 24 is inserted into the locking hole 25. The biasing spring 30 applies a permanent moment on the swivel plate 27, biasing it to rotate towards the latch 15. The latch 15 however is designed such that, upon being in the unlatched state, such as shown in the figures 4 and 5, the latch 15 counteracts the biasing force on the swivel plate 27, because the latch 15 comprises an extending part blocking the rotation of the swivel plate 27 when the latch 15 is in the unlatched state. The swivel plate 27 is therefore not allowed to rotate to a position where the swivel plate 27 is positioned underneath of the locking pin 24. The swivel plate 27 is thus forced to remain in a state referred to as an open state, meaning not being between the locking hole 25 and the locking pin 24. It is furthermore noted that the locking pin 24 is biased against the latch 15 surface, therefore additionally blocking the movable plate 27 from being brought into the closed state wherein the movable plate is between the locking pin 24 and the locking hole 25. Figure 10 shows the stage of the patient support assembly 1 of figures 4 and 5, in the top stage box symbolizing the stage «unlatched and ready to mount». As illustrated in figure 10 as a transition from the top stage box to the bottom right stage box, the latch actuator is automatically brought from an actuated state into a non-actuated state, and simultaneously the locking device 24 is brought from an unlocked state to a locked state, upon the latch 15 reaching the latched state, whereby the latch 15 is moved to a position where the locking hole 25 is positioned underneath of the locking pin 24, allowing the biasing force on the locking pin 24 to insert the locking pin 24 into the locking hole 25. The latch locking device 24 in the locked state prevents the latch 15 from being brought back into the unlatched state, as symbolized by the crossed transition from the bottom right stage box to the top stage box in figure 10. Upon the latch 15 being brought into the latched state, the extending part of the latch 15 stops blocking the rotation of the swivel plate 27. However, the latch 15, specifically the extending part of the latch 15, the locking device 24 and the swivel plate 27 are designed such that the locking pin 24 is inserted into the locking hole 25 before the swivel plate 27 can swivel between the locking pin 24 and the locking hole 25 upon the latch reaching the latched state. Therefore, the swivel plate 27 remains in the open state, as the biasing force on the swivel plate 27 is counteracted by the swivel plate 27 contacting the locking pin 24 inserted into the locking hole 25. In particular, whilst the latch 15 being brought into the latched state, the extending part of the latch 15 gradually loses its blocking effect on the movable plate 27. During this transition however, the locking device 24, in particular the locking pin 24 is biased to push against the latch 15 surface and thus gradually increases its blocking effect on the movable plate 27 until the point where the locking pin is inserted, for example falls into the locking hole 25 thereby guaranteeing that the movable plate remains in the opened state. Upon the latch locking device 24 being in the locked state, and the latch 15 being in the latched state, the patient support assembly 1 is in the stage as symbolized by the bottom right stage box in figure 10, referred to as «Latched and locked». The «Latched and locked» stage of the patient support assembly 1 is illustrated in the figures 6 and 7. Finally, when it is desired that the extension panel 6 is removed from the patient support panel 7, the latch actuator 26 is actuated, causing the locking pin 24 to be lifted out of the locking hole 25 and up to a height whereby locking pin 24 is no longer able to exert a counteracting force on the swivel plate 27, thereby allowing the swivel plate 27 to swivel, due to the biasing force, in a position between the locking hole 25 and the locking pin 24. This position is referred to as the closed position of the swivel plate 27. Upon the locking device 24 being unlocked, i.e. the locking pin 24 removed from the locking hole 25, and the latch 15 being in the latched state, the patient support assembly is said to be in the stage of «Latched and ready to remove» as symbolized by the bottom left stage box in figure 10. The patient support assembly 1 in this stage is illustrated in the figures 8 and 9. The swivel plate 27 being in the closed position prevents the latch actuator 26 and by consequence the locking device 24 from being brought back into respectively the non-actuated state and the locked state, as symbolized by the crossed transition from the bottom left stage box to the bottom right stage box in figure 10. It is noted that in this stage, the user is no longer required to exert a force on the latch actuator 26 in order to maintain it in the actuated state, despite the biasing force acting on the latch actuator 26, because the latch actuator 26 is biased to push, for example due to gravity or a spring, against the surface of the movable plate 27 in its closed position. Finally, the user such as the nurse or the technical assistant translates the extension panel 6 away from the patient support panel 7 thereby bringing the latch 15 from the latched state to the unlatched state. The transition is symbolized by the arrow from the bottom left stage box to the top stage box in figure 10. The latch being biased to be in the unlatched state by the permanent action of biasing spring 29, assists in the releasing of the attachment member 16 from the abutment part 21 and the latching part 22. The latch 15 being brought into the unlatched state, causes the extension part of the latch 15 to counteract the biasing force applied on the swivel plate 27 such as to bring the swivel plate 27 from the closed state into the opened state i.e. no longer in a position between the locking pin 24 and the locking hole 25 and specifically in a position no longer underneath the locking pin 24. The latch 15 having moved from the latched state to the unlatched state has thereby also moved the position of the locking hole 25 away from the position underneath the locking pin 24, Under the influence of the biasing force, the locking pin 24 is thus pushed against the latch 15 at a position away from the locking hole 25 and thus in an unlocked state. The latch actuator 26 is thereby also forced to remain in the actuated state, as the locking device 24 cannot be locked. As noted above, the latch 15, specifically the extending part of the latch 15, the locking device 24 and the swivel plate 27 are designed such that the locking pin 24 is inserted into the locking hole 25 before the swivel plate 27 can swivel between the locking pin 24 and the locking hole 25 upon the latch 15 reaching the latched state, in particular due to the locking device 24, in particular the locking pin being biased to push against the latch 15 surface, thus preventing the movable plate 27 to enter the closed state where it would be positioned between the latch 15, and a fortiori the locking hole 25, and the locking pin 24. This is symbolized as the crossed transition between the top stage box and the bottom left stage box in figure 10.

## Claims

1. A patient support assembly (1) for medical use, such as for radiation therapy or radiation diagnostics, comprising a joint assembly (2) releasably connecting an extension panel (6) to a patient support panel (7) mounted on a pedestal, the joint assembly (2) comprising the extension panel (6) and a second body member (4), the extension panel comprising a first body member (3), wherein the first body member (3) is coupled to the extension panel (6) and wherein the second body member (4) is coupled to the patient support panel (7), the first body member (3) comprising one of a cavity member (5) delimited by an upper cavity member surface (31) and a lower cavity member surface (32) extending in an engagement direction, the length direction of the panels, between a cavity member closed end (8) and a cavity member open end (9), and a tab member (10) delimited by an upper tab member surface (11) and a lower tab member surface (12) extending in the engagement direction between a tab member base end (13) and a tab member free end (14), the second body member (4) comprising the other one of the cavity member (5) and the tab member (10),
the first body member (3) further comprising one of a latch (15) and an attachment member (16), and the second body member (4) comprising the other one of the attachment member (16) and the latch (15), wherein the first body member (3) is arranged to be brought into engagement with the second body member (4), by moving the cavity member (5) with respect to the tab member (10) and by latching the attachment member (16) with the latch (15), wherein the cavity member (5) is moveable over the tab member (10) by means of a translation movement in the engagement direction up to a locking position where the latch (15) latches on the attachment member (16), wherein the latch (15) comprises an abutment part (21) and a latching part (22), wherein, upon the cavity member (5) being translated over the tab member (10) towards the tab member base end (13), the attachment member (16) abuts the abutment part (21) causing the latch (15) to switch from an unlatched state to a latched state wherein the latching part (22) latches on to the attachment member (16), **characterized in that** the latch (15) is biased to be in the unlatched state such that upon the cavity member (5) being translated over the tab member (10) towards the tab member free end (14), the attachment member (16) releases the abutment part (21) causing the latch (15) to switch from the latched state to the unlatched state.

2. The patient support assembly (1) according to the preceding claim, wherein the first body member (3) comprises the cavity member (5), and wherein the upper tab member surface (11) supports the upper cavity member surface (31), when the cavity member (5) is translated over the tab member (10).

3. The patient support assembly (1) according to any one of the preceding claims, wherein the tab member (10) and cavity member (5) comprise a part of finite length (19, 20) wherein the upper tab member surface (11) lies parallel to the lower tab member surface (12) and wherein the upper cavity member surface (31) lies substantially parallel to the lower cavity member surface (32), and wherein, upon the first body member (3) being engaged with the second body member (4), the upper cavity member surface (31) and the lower cavity member surface (32) are adjacent to respectively the upper tab member surface (11) and lower tab member surface (12), along a segment of the part of finite length (19,20), the supporting segment (33, 34), such as to create a close sliding fit.

4. The patient support assembly (1) according to any one of the preceding claims, wherein the body member comprising the latch (15), comprises a latch locking device (24) which is brought from an unlocked state to a locked state when the latch (15) is brought into the latched state, and wherein the latch locking device (24) in the locked state prevents the latch (15) from being brought into the unlatched state.

5. The patient support assembly (1) according to the preceding claim, wherein one of the first body member (3) and second body member (4) comprises a latch actuator (26) configured to, upon actuation, bring the latch locking device (24) from the locked state into the unlocked state.

6. The patient support assembly (1) according to any one of the preceding claims 4 -5, wherein the body member comprising the latch (15) comprises a moveable plate switchable between an opened state and a closed state, wherein the moveable plate (27) in the closed state prevents the latch locking device (24) from being brought in the locked state, and wherein the moveable plate (27) is brought into the closed state when the latch locking device (24) is brought in the unlocked state and the latch (15) is in the latched state.

7. The patient support assembly (1) according to the preceding claim, wherein the moveable plate (27) is biased to be in the closed stated and wherein both the latch locking device (24) being in the locked state as well as the latch (15) being in the unlatched state prevent the moveable plate (27) from being brought into the closed state.

8. The patient support assembly (1) according to any one of the preceding claims 6 - 7, wherein the moveable plate (27) in the closed state is brought into the opened state when the latch (15) is brought into the unlatched state.

9. The patient support assembly (1) according to any one of the preceding claims, wherein the extension panel (6) is a radiolucent panel.

## Patentansprüche

1. Eine Patientenlagerungsanordnung (1) zur medizinischen Anwendung, wie für Strahlentherapie oder Strahlendiagnose, welche eine Verbindungsanordnung (2) umfasst, die eine Verlängerungsplatte (6) abnehmbar mit einer Patientenlagerungsplatte (7) verbindet, welche auf einem Sockel montiert ist, wobei die Verbindungsanordnung (2) die Verlängerungsplatte (6) und ein zweites Hauptelement (4) umfasst, wobei die Verlängerungsplatte ein erstes Hauptelement (3) umfasst, wobei das erste Hauptelement (3) an die Verlängerungsplatte (6) gekoppelt ist und wobei das zweite Hauptelement (4) an die Patientenlagerungsplatte (7) gekoppelt ist, wobei das erste Hauptelement (3) ein Hohlraumelement (5), begrenzt durch eine obere Hohlraumelementoberfläche (31) und eine untere Hohlraumelementoberfläche (32), die sich in eine Eingreifrichtung, die Längsrichtung der Platten, zwischen einem geschlossenen Hohlraumelementende (8) und einem offenen Hohlraumelementende (9) ausdehnen, und ein Laschenelement (10) umfasst, begrenzt durch eine obere Laschenelementoberfläche (11) und eine untere Laschenelementoberfläche (12), die sich in die Eingreifrichtung zwischen einem Laschenelementbasisende (13) und einem freien Laschenelementende (14) ausdehnen, wobei das zweite Hauptelement (4) das andere Hohlraumelement (5) und das Laschenelement (10) umfasst,
wobei das erste Hauptelement (3) ferner ein Element von Sperrklinke (15) und Befestigungselement (16) umfasst, und das zweite Hauptelement (4) das andere Element von Befestigungselement (16) und Sperrklinke (15) umfasst, wobei das erste Hauptelement (3) angeordnet ist, um zum Eingreifen mit dem zweiten Hauptelement (4) gebracht zu werden, indem das Hohlraumelement (5) in Bezug auf das Laschenelement (10) bewegt wird und indem das Befestigungselement (16) mit der Sperrklinke (15) eingeklinkt wird, wobei das Hohlraumelement (5) über das Laschenelement (10) mittels einer Schiebebewegung in die Eingreifrichtung bis zu einer Verriegelungsposition beweglich ist, in der die Sperrklinke (15) in das Befestigungselement (16) einklinkt, wobei die Sperrklinke (15) ein Anschlagteil (21) und ein Einklinkteil (22) umfasst, wobei das Befestigungselement (16), wenn das Hohlraumelement (5) über das Laschenelement (10) hin zum Laschenelementbasisende (13) geschoben wird, an das Anschlagteil (21) anliegt, wodurch die Sperrklinke (15) von einem gelösten Zustand in einen eingeklinkten Zustand wechselt, in dem das Einklinkteil (22) in das Befestigungselement (16) einklinkt, **dadurch gekennzeichnet, dass** die Sperrklinke (15) vorgespannt ist, um im gelösten Zustand zu sein, sodass das Befestigungselement (16), wenn das Hohlraumelement (5) über das Laschenelement (10) hin zum freien Laschenelementende (14) geschoben wird, das Anschlagteil (21) freigibt, wodurch die Sperrklinke (15) vom eingeklinkten Zustand in den gelösten Zustand wechselt.

2. Die Patientenlagerungsanordnung (1) nach dem vorigen Anspruch, wobei das erste Hauptelement (3) das Hohlraumelement (5) umfasst, und wobei die obere Laschenelementoberfläche (11) die obere Hohlraumelementoberfläche (31) unterstützt, wenn das Hohlraumelement (5) über das Laschenelement (10) geschoben wird.

3. Die Patientenlagerungsanordnung (1) nach irgendeinem der vorigen Ansprüche, wobei das Laschenelement (10) und das Hohlraumelement (5) einen Teil endlicher Länge (19, 20) umfassen, wobei die obere Laschenelementoberfläche (11) parallel zur unteren Laschenelementoberfläche (12) liegt und wobei die obere Hohlraumelementoberfläche (31) im Wesentlichen parallel zur unteren Hohlraumelementoberfläche (32) liegt, und wobei die obere Hohlraumelementoberfläche (31) und die untere Hohlraumelementoberfläche (32), wenn das erste Hauptelement (3) mit dem zweiten Hauptelement (4) verbunden wird, an respektive die obere Laschenelementoberfläche (11) und die untere Laschenelementoberfläche (12) entlang eines Abschnitts des Teils endlicher Länge (19, 20), des unterstützenden Abschnitts (33, 34), anliegen, um so einen eng anliegenden Gleitsitz zu erzeugen.

4. Die Patientenlagerungsanordnung (1) nach irgendeinem der vorigen Ansprüche, wobei das Hauptelement, welches die Sperrklinke umfasst, eine Sperrklinkenverriegelungsvorrichtung (24) umfasst, die von einem entriegelten Zustand in einen verriegelten Zustand gebracht wird, wenn die Sperrklinke (15) in den eingeklinkten Zustand gebracht wird, und wobei die Sperrklinkenverriegelungsvorrichtung (24) im verriegelten Zustand verhindert, dass die Sperrklinke (15) in den gelösten Zustand gebracht wird.

5. Die Patientenlagerungsanordnung (1) nach dem vorigen Anspruch, wobei ein Element des ersten Hauptelements (3) und des zweiten Hauptelements (4) einen Sperrklinkenbetätiger (26) umfasst, konfiguriert, um, bei Betätigung, die Sperrklinkenverriegelungsvorrichtung (24) aus dem verriegelten Zustand in den entriegelten Zustand zu bringen.

6. Die Patientenlagerungsanordnung (1) nach irgendeinem der vorigen Ansprüche 4 bis 5, wobei das Hauptelement, welches die Sperrklinke (15) umfasst, eine bewegliche Platte umfasst, die zwischen einem offenen Zustand und einem geschlossenen Zustand wechselbar ist, wobei die bewegliche Platte (27) im geschlossenen Zustand verhindert, dass die Sperrklinkenverriegelungsvorrichtung (24) in den verriegelten Zustand gebracht wird, und wobei die bewegliche Platte (27) in den geschlossenen Zustand gebracht wird, wenn die Sperrklinkenverriegelungsvorrichtung (24) in den entriegelten Zustand gebracht wird und die Sperrklinke (15) im eingeklinkten Zustand ist.

7. Die Patientenlagerungsanordnung (1) nach dem vorigen Anspruch, wobei die bewegliche Platte (27) vorgespannt ist, um im geschlossenen Zustand zu sein, und wobei sowohl die Sperrklinkenverriegelungsvorrichtung (24), die im verriegelten Zustand ist, als auch die Sperrklinke (15), die im gelösten Zustand ist, verhindern, dass die bewegliche Platte (27) in den geschlossenen Zustand gebracht wird.

8. Die Patientenlagerungsanordnung (1) nach irgendeinem der vorigen Ansprüche 6 bis 7, wobei die bewegliche Platte (27) im geschlossenen Zustand in den geöffneten Zustand gebracht wird, wenn die Sperrklinke (15) in den gelösten Zustand gebracht wird.

9. Die Patientenlagerungsanordnung (1) nach irgendeinem der vorigen Ansprüche, wobei die Verlängerungsplatte (6) eine strahlendurchlässige Platte ist.

## Revendications

1. Ensemble de support de patient (1) pour un usage médical, par exemple pour la radiothérapie ou le diagnostic radiologique, comprenant un ensemble d'articulation (2) reliant de manière amovible un panneau d'extension (6) à un panneau de support de patient (7) monté sur un socle, l'ensemble d'articulation (2) comprenant le panneau d'extension (6) et un second élément de corps (4), le panneau d'extension comprenant un premier élément de corps (3), dans lequel le premier élément de corps (3) est accouplé au panneau d'extension (6) et dans lequel le second élément de corps (4) est accouplé au panneau de support de patient (7), le premier élément de corps (3) comprenant un parmi un élément de cavité (5) délimité par une surface d'élément de cavité supérieure (31) et une surface d'élément de cavité inférieure (32) s'étendant dans une direction de mise en prise, le sens de la longueur des panneaux, entre une extrémité fermée d'élément de cavité (8) et une extrémité ouverte d'élément de cavité (9), et un élément de languette (10) délimité par une surface d'élément de languette supérieure (11) et une surface d'élément de languette inférieure (12) s'étendant dans la direction de mise en prise entre une extrémité de base d'élément de languette (13) et une extrémité libre d'élément de languette (14), le second élément de corps (4) comprenant l'autre parmi l'élément de cavité (5) et l'élément de languette (10),
le premier élément de corps (3) comprenant en outre un parmi un verrou (15) et un élément de fixation (16), et le second élément de corps (4) comprenant l'autre parmi l'élément de fixation (16) et le verrou (15), dans lequel le premier élément de corps (3) est agencé pour être mis en prise avec le second élément de corps (4), en déplaçant l'élément de cavité (5) par rapport à l'élément de languette (10) et en verrouillant l'élément de fixation (16) avec le verrou (15), dans lequel l'élément de cavité (5) est mobile sur l'élément de languette (10) au moyen d'un mouvement de translation dans la direction de mise en prise jusqu'à une position de verrouillage où le verrou (15) se verrouille sur l'élément de fixation (16), dans lequel le verrou (15) comprend une partie de butée (21) et une partie de verrouillage (22), dans lequel, lorsque l'élément de cavité (5) effectue une translation sur l'élément de languette (10) vers l'extrémité de base d'élément de languette (13), l'élément de fixation (16) vient buter contre la partie de butée (21), amenant le verrou (15) à passer d'un état déverrouillé à un état verrouillé dans lequel la partie de verrouillage (22) se verrouille sur l'élément de fixation (16), **caractérisé en ce que** le verrou (15) est dévié pour être dans l'état déverrouillé de sorte que lorsque l'élément de cavité (5) effectue une translation sur l'élément de languette (10) vers l'extrémité libre d'élément de languette (14), l'élément de fixation (16) libère la partie de butée (21), amenant le verrou (15) à passer de l'état verrouillé à l'état déverrouillé.

2. Ensemble de support de patient (1) selon la revendication précédente, dans lequel le premier élément de corps (3) comprend l'élément de cavité (5), et dans lequel la surface d'élément de languette supérieure (11) supporte la surface d'élément de cavité supérieure (31), lorsque l'élément de cavité (5) effectue une translation sur l'élément de languette (10).

3. Ensemble de support de patient (1) selon l'une quelconque des revendications précédentes, dans lequel l'élément de languette (10) et l'élément de cavité (5) comprennent une partie de longueur finie (19, 20) dans laquelle la surface d'élément de languette supérieure (11) est parallèle à la surface d'élément de languette inférieure (12) et dans laquelle la surface d'élément de cavité supérieure (31) est sensiblement parallèle à la surface d'élément de cavité inférieure (32), et dans lequel, lorsque le premier élément de corps (3) est en prise avec le second élément de corps (4), la surface d'élément de cavité supérieure (31) et la surface d'élément de cavité inférieure (32) sont adjacentes respectivement à la surface d'élément de languette supérieure (11) et à la surface d'élément de languette inférieure (12), le long d'un segment de la partie de longueur finie (19, 20), le segment de support (33, 34), de manière à créer un ajustement coulissant serré.

4. Ensemble de support de patient (1) selon l'une quelconque des revendications précédentes, dans lequel l'élément de corps comprenant le verrou (15) comprend un dispositif de verrouillage de verrou (24) qui est mis d'un état déverrouillé à un état verrouillé lorsque le verrou (15) est mis dans l'état verrouillé, et dans lequel le dispositif de verrouillage de verrou (24) dans l'état verrouillé empêche le verrou (15) d'être mis dans l'état déverrouillé.

5. Ensemble de support de patient (1) selon la revendication précédente, dans lequel un parmi le premier élément de corps (3) et le second élément de corps (4) comprend un actionneur de verrou (26) configuré pour, lors de l'actionnement, mettre le dispositif de verrouillage de verrou (24) de l'état verrouillé à l'état déverrouillé.

6. Ensemble de support de patient (1) selon l'une quelconque des revendications précédentes 4-5, dans lequel l'élément de corps comprenant le verrou (15) comprend une plaque mobile qui peut passer dans un état ouvert et dans un état fermé, dans lequel la plaque mobile (27) dans l'état fermé empêche le dispositif de verrouillage de verrou (24) d'être mis dans l'état verrouillé, et dans lequel la plaque mobile (27) est mise dans l'état fermé lorsque le dispositif de verrouillage de verrou (24) est mis dans l'état déverrouillé et le verrou (15) est dans l'état verrouillé.

7. Ensemble de support de patient (1) selon la revendication précédente, dans lequel la plaque mobile (27) est déviée pour être dans l'état fermé et dans lequel le dispositif de verrouillage de verrou (24) dans l'état verrouillé ainsi que le verrou (15) dans l'état déverrouillé empêchent tous deux la plaque mobile (27) d'être mise dans l'état fermé.

8. Ensemble de support de patient (1) selon l'une quelconque des revendications précédentes 6-7, dans lequel la plaque mobile (27) dans l'état fermé est mise dans l'état ouvert lorsque le verrou (15) est mis dans l'état déverrouillé.

9. Ensemble de support de patient (1) selon l'une quelconque des revendications précédentes, dans lequel le panneau d'extension (6) est un panneau radiotransparent.
